# EUROPEAN PATENT APPLICATION

(11) **EP 1 764 076 A2**
(43) Date of publication of application: **21.03.2007**
(21) Application number: 06019270.5
(22) Date of filing: 14.09.2006
(51) Int. Cl.: A61K 8/02, A61Q 19/10, A61Q 19/00, A61K 8/34, A61K 8/92

(54) **Hygienic and cosmetic compositions for treating atopical dermatitis**

(30) Priority: 16.09.2005 IT MI20051718
(71) Applicant: Betafarma S.p.A., 20090 Cesano Boscone (IT)
(72) Inventor: Tallia, Ettore, 20090 Cesano Boscone (Milano) (IT)
(74) Representative: Cicogna, Franco

(57) **Abstract**

A series of hygienic cosmetic products which, without a particular pharmacological activity, are adapted to be used according to a differentiated functional and specific use, on skins having symtoms related to atopic dermatitis, are herein disclosed. The compositions reduce pruriginous feelings and related scratching stimulation, while holding a proper physiologic hydration level of the skin, by adjusting the so-called "perspiratio insensibilis", while preventing the skin from being imbibed by a water excess, thereby minimizing eliciting factors and irritating reactions of an atopic skin. The hygienic composition according to the invention are characterized in that the water which is present in their formulations is mostly bound with a low value of "free water".

## Description

### BACKGROUND OF THE INVENTION

The present invention relates to hygienic and cosmetic compositions for processing atopical dermatites.

More specifically, the present invention relates to a series of hygienic cosmetic products which, while being devoid of any particular pharmacologic activities, are adapted to be used, in a differentiated functional and specific manner, on skins showing symptoms related to atopical dermatites.

As is known, atopia may be defined as a genetical trend to an exaggerated reactivity of the skin and mucosas in response to environmental stimula.

Actually, atopic dermatitis is a chronic skin pathology, of a recursive type, which statically starts at an early childhood age, and frequently continues to the adult age.

It is considered as the most common and irritating problem of a child skin, starting from an age of about six months.

The impact of the above mentioned disease in children is continuously increasing: from a 3% rate, immediately after the Second World War, to a rate of 10% today, since child epidermis is frequently exposed to polluting and irritating substances.

Moreover, said disease has been additionally found to be related to a breast-feeding decrease of newborns.

The skin zones which are usually affected by atopical dermatitis, such as the face, flexible areas inside the knees and elbows, the neck area, depend on the patient age.

The disease symptoms and signs appear, at the start, as a redness and chapping of the cheeks. Then said symptoms can diffuse to the overall face, breast, scalp, hands, always with a strong itch.

The most typical characteristics of age related atopic dermatitis are shown in the following table:

| **AGE** | **AFFECTED SKIN ZONE** | **MS SYMPTOMS** |
|---|---|---|
| 2 months | Face, breast | Redness, vesicles, dry skin, itch |
| 2 years | Scalp, neck, limb stretching surfaces | Injuries, Edema, Erythema |
| 2 - 4 years | Neck, wrists, elbow, knees | Dry thickened, hyper-pigmented plaques |
| 12 - 20 years | Hands, limb stretching surfaces | Dry skin, Hyperpigmented plaques |

Fortunately, the above mentioned dermatitis symptoms start to spontaneously improve and revert, in a lot of cases, from an age of two to four years and can also disappear as the child nears an adult age.

Atopic dermatitis has a genetic basis.

In fact, a comparatively high rate of cases presents an atopic family history, but its expression and seriousness are modified according to a broad spectrum of hexogenous factors.

On the other hand, the above mentioned skin pathology cannot be properly diagnosed based on clinical criteria, since no specific safe and reliable laboratory tests are at present available; thus it is necessary to properly consider several elements aggravating this disease.

In particular, patients affected by atopical dermatitis may be very sensitive to a comparatively low concentration of irritating substances (which, on a regular skin, would not cause any objectable reactions), such as soaps, solvents, preserving and microbiocidal materials, perfuming substances, synthetic dyes, glycol moisturizing substances, cleansing anionic substances, or SLS-SLES, which may cause burning, itch or redness.

Allergenic substances, on the other hand, such as plants or animal proteins (glycolic or vegetal oil extracts, flower or herb functional principles, milk derivatives and son on) can also aggravate atopic dermatitis.

With respect to the skin dehydration, it must be pointed out that the corneal layer, in a patient affected by atopical dermatitis, has a moisture contents much less than that of a regular skin.

An improvement of the skin hydration can be obtained by non-pharmacologic means, for example a systematic use of emollient and hydrating compositions.

On the other hand, for decreasing itch symptoms, the affected persons must live in low temperature and moisture contents environments.

Another aspect to be considered in patients affected by atopical dermatitis is the bathing made.

Some dermatologists oppose to daily bathes, since they think that a repeated immersion into water would quickly dry the skin, and generate microcracks therein, to favor an inlet of skin allergenic and irritating organisms.

On the other hand, other dermatologists state that bathes, taken in a suitable manner in tepid water, for a short duration and two or three times for week, (without using soaps, susceptible to dry the skin), would moisturize the corneal layer and remove allergenic and irritating substances therefrom.

For atopical patient, water dispersible oils leaving emollient and lubricating substance traces on the skin would be suitable.

Alternately, it would be also possible to use specifically designed amphoteric foaming substances, of a very delicate nature, with a low free-water amount, and added with oat meal or starches having satisfactory anti-itching properties.

After bathing, and a slight drying, it is necessary to immediately apply on dermatitis affected skin areas an anhydrous ointment, with a lenitive and moisturizing and protecting-emollient action.

### SUMMARY OF THE INVENTION

Accordingly, the aim of the present invention is to provide a series of skin processing compositions, which, while being devoid of a particular pharmacologic activity, are adapted to greatly reduce symptoms caused by atopic dermatitis.

Within the scope of the above mentioned aim, a main object of the invention is to provide such a composition adapted to decrease itch feelings and consequent scratching stimulations.

Another object of the invention is to provide such a composition which is adapted to hold a skin moisturizing proper and physiologic level, while adjusting the "perspiration insensibilis" and preventing skin from being imbibed by an excessive amount of water.

Yet another object of the invention is to provide such a composition adapted to minimize atopical dermatitis eliciting factors and consequent atopical skin irritation reactions.

According to one aspect of the present invention, the above mentioned aim and objects, as well as yet other objects, which will become more apparent hereinafter, are achieved by hygienic compositions, for treating atopic dermatitis, characterized in that said compositions have a composition formula including water which is substantially bound with a low free water value.

The subject compositions may be an anhydrous type, such as a water dispersible cleansing oil, or a lenitive-protective hydrating ointment, or any other desired types, such as a foaming cleansing substance, a shampoo, or a fluid protective hydrating cream.

The Applicant has found that the free water value of all the above mentioned compositions must be less than 0.7.

Actually, the above mentioned chemical-physical parameter is indicative of the amount of water which is intimately bound in the composition formulation (independently from the concentration rate of the composition) and which can be hardly released to the skin.

In other words, the above mentioned free water parameter is indicative of the "active" portion of the total water amount affecting the physical, chemical, electric properties and microbiologic undegradability of the composition.

The following illustrative, but not limitative, examples show further details of preferred embodiments of the invention.

In this connection it should be apparent that said examples are susceptible to several modification and variations, all of which will come within the scope of the invention as herein disclosed and claimed.

### Example 1

### Foaming cleansing shampoo - bath composition

| Components | % w/w | Function |
|---|---|---|
| Polyalcohols | 55-80 | moisturizing, hydrating |
| Amphoteric foaming materials | 5-15 | cleansing |
| Sorbitanesters | 0-5 | emulsifying |
| Starch | 3-8 | refreshing |
| Magnesium sulphate | 0.1-1 | protecting |
| E Vitamine | 0.1-0.5 | anti-radical |
| Purified water | q.s. to 100 | |

### Example 2

### Water dispersible cleansing oil

| Components | % w/w | Function |
|---|---|---|
| Petrolatum oils | 90-95 | protecting-moisturizing |
| Aliphatic alcohols P.O.E. | 2-5 | cleansing |
| Aliphatic esters | 0.5-3 | emollient |
| E-Vitamin | 0.05-0.3 | antiradical |

### Example 3

### Lenitive-protective moisturizing oinment

| Components | % w/w | Function |
|---|---|---|
| Petrolatum oil F.U. | 60-90 | protecting-moisturizing |
| Styrene copolymers | 2-5 | stabilizing |
| Petrolatum F.U. | 10-40 | protecting-emollient |
| Ozochemite | 1-3 | gelifying |
| Zinc oxide | 2-10 | absorbing |
| Starch | 2-5 | refreshing |
| E-Vitamin | 0.05-0.3 | antiradical |

### Example 4

### Fluid moisturizing-protective cream

| Components | % w/w | Function |
|---|---|---|
| Emulsifying W/O agent | 1-4 | emulsifying |
| Gelifying wax | 1-5 | reology modifier |
| Petrolatum oil F.U. | 15-30 | moisturizing-protecting |
| Aliphaticesters | 10-20 | emollient |
| E-Vitamin | 0.05-0.4 | antiradical |
| Polyalcohols | 15-50 | moisturizing-hydrating |
| Starch | 2-5 | refreshing |
| Sulphate magnesio | 1-3 | protecting |
| Purified water | q.s. to 100 | |

It has been found that the invention fully achieves the intended aim and objects.

In fact, the invention provides skin processing or treating compositions adapted to greatly reduce itch feelings and a consequent scratching stimulation, while holding a proper and physiologic level of skin hydrating, and adjusting the perspiration insensibilis; moreover, skin is not imbibed by an excess amount of water, thereby minimizing dermatitis disease eliciting factors and consequent irritating reactions on an atopical skin.

Thus, as the subject compositions are applied on an atopic skin, they do not release water on skin, owing to their anhydrous nature and have a very reduced free water contents.

Moreover, the hygienic compositions according to the present invention do not comprise soaps, solvents, preserving or microbiocidal substances, perfumes, dyes, glycols, anionic cleansing substances such as SLS-SLES, glycole extracts and oil plant extracts, and milk derivatives.

## Claims

1. A hygienic composition for treating atopical dermatitis, **characterized in that** said hygienic composition has a water contents comprising substantially fully bound water with a free water value less than 0.7.

2. A composition according to claim 1, **characterized in that** said composition comprises a foaming cleansing shampoo-bath compound including the following components, in a by weight rate: Polyalcohols 55-80%, Amphoteric foaming agents 5-15%, Sorbitanesters 0-5%, Starch 3-8%, Sulphate magnesium 0.1-1%. E-Vitamin 0.1-0.5%, Purified water q.s. to 100.

3. A composition according to claim 1, **characterized in that** said composition comprises a water soluble cleansing oil comprising the following components, in a by rate weight: Petrolatum oil 90-95%, P.O.E. Aliphatic alcohols 2-5%, Aliphatic esters 0.5-3%, E-Vitamin 0.05-0.3%.

4. A composition according to claim 1, **characterized in that** said composition comprises a lenitive protective hydrating ointment including the following components, in a by weight rate: F.U. Petrolatum oil 60-90%, Styrene copolymer 2-5%, F.U. Petrolatum 10-40%, Ozochenite 1-3%, Zinc oxide 2-10%, Starch 2-5%, E-Vitamin 0.05-0.3%.

5. A composition according to claim 1, **characterized in that** said composition comprises a fluid hydrating protective cream comprising the following components, in a by weight rate: W/O emulsifier 1-4%, gelifying waxes 1-5%, F.U. Petrolatum oil 15-30%, Aliphatic esters 10-20%, E-Vitamin 0.05-0.4%, Polyalcohols 15-50%, Starch 2-5%, Magnesium sulphate 1-3%, Purified water q.b. to 100.
